# EUROPEAN PATENT APPLICATION

(11) **EP 1 333 089 A1**
(43) Date of publication of application: **06.08.2003**
(21) Application number: 01976852.2
(22) Date of filing: 26.10.2001
(51) Int. Cl.: C12N 15/10, C12Q 1/68

(54) **METHOD OF SYNTHESIZING SINGLE-STRANDED NUCLEIC ACID**

(30) Priority: 27.10.2000 JP 2000328219
(71) Applicant: Eiken Kagaku Kabushiki Kaisha, Bunkyo-ku, Tokyo 113-8408 (JP)
(72) Inventor: NAGAMINE, K., c/o NASU KOJO, EIKEN KAGAKU K.K., Otawara-shi, Tochigi 324-003 (JP); HASE, Tetsu, c/o Japan Genome Solutions Inc., Hachioji-shi, Tokyo 192-0031 (JP); NOTOMI, T., c/o NASU KOJO, EIKEN KAGAKU K.K., Otawara-shi, Tochigi 324-003 (JP)
(74) Representative: Schrell, Andreas, Dr.
(86) International application number: JP0109452
(87) International publication number: WO02034907

(57) **Abstract**

This invention relates to a method for selectively and efficiently synthesizing one of the sense or antisense strands of double-stranded nucleic acid. The method for synthesizing single-stranded nucleic acid according to this invention comprises the following steps of:
1) cleaving, with a restriction enzyme, double-stranded DNA having a restriction enzyme recognition sequence at a portion closer to the 5' side than the target sequence in such a manner that
   a) a fragment having an overhanging 3' terminus is formed, and
   b) base sequences of the single-stranded regions of the fragments after the cleavage differ from each other,
2) to the single-stranded region of the DNA fragment that was cleaved with the restriction enzyme, annealing a primer having a base sequence complementary to the region on at least its 3' terminus; and
3) synthesizing a nucleic acid by a strand displacement-type polymerase starting from the 3' terminus of the primer.

Further, a single-stranded nucleic acid can be more efficiently synthesized by amplifying the double-stranded DNA having the restriction enzyme recognition sequence by the LAMP reaction.

## Description

### Technical Field

The present invention relates to a method for synthesizing single-stranded DNA, and more particularly to a method for selectively synthesizing one of the sense or antisense strands of double-stranded DNA.

### Background Art

The most common method for preparing single-stranded DNA that is used as a probe in the hybridization assay is carried out by denaturing double-stranded DNA by heat or alkali. With this method, however, complementary strands are present in the product, and thus, complementary strands are rebound to each other under conditions for forming a double strand. This sometimes deteriorates the hybridization efficiency. Also known is a method for preparing single-stranded DNA through cloning by utilizing a single-stranded phage such as M13, although this method is costly and time-consuming.

A short single-stranded DNA sequence comprising approximately several dozen bases can be chemically synthesized without difficulty. In contrast, if a long single-stranded DNA sequence having more than 100 bases is chemically synthesized, the yield and the accuracy of base sequences deteriorate. Although the T7 RNA polymerase can synthesize single-stranded RNA using DNA as a template, the template DNA needs a promoter sequence which is recognized by the aforementioned enzyme.

In contrast, WO 99/09211 discloses a method for amplifying a target sequence on the first strand of a double-stranded nucleic acid. In this method, the double-stranded nucleic acid is cleaved using a restriction enzyme, and with this cleavage, the 5' terminus of the target sequence on the first strand is cleaved so as to form an overhanging 3' terminal region on the second strand. The extension reaction is then carried out using a primer complementary to the 3' terminus of the second strand and a strand displacement-type polymerase and employing the second strand as a template, thereby amplifying the target sequence. In this method, a restriction site is also regenerated simultaneously with the synthesis of the target region by a primer in principle. However, the target region should have the restriction site at the 5' terminus when the reaction is initiated, and the provision of the recognition site is not sufficiently disclosed. Also, this method is an improved version of the Strand Displacement Amplification (SDA) method [Proc. Natl. Acad. Sci. USA, 89, 392-396; 1992] [Nucleic Acid. Res., 20, 1691-1696; 1992], which originally aimed at amplifying double-stranded DNA. Accordingly, when the sequences of the overhanging portions of each of the 3' sides created by cleavage are identical to each other (palindrome sequences), both the sense and antisense strands are synthesized, even with the use of only one primer. Specifically, this method does not always provide single-stranded DNA.

The SDA method is briefly described. The SDA method is a method utilizing a special DNA polymerase. When complementary strand is synthesized starting from a primer that is complementary to the 3' side of a certain base sequence and a double-stranded region is present on the 5' side, the complementary strand is synthesized so as to displace one strand of the double-stranded region. In the SDA method, previous insertion of the restriction enzyme recognition sequence in the annealed sequence by a primer can allow the omission of the step of temperature variation that is essential in PCR. Specifically, the nick, which is generated by a restriction enzyme, imparts the 3'-OH group as a starting point of complementary strand synthesis, and strand displacement synthesis is performed therefrom. Thus, the previously synthesized complementary strand is freed as a single-strand and reused as a template for the next complementary strand synthesis. In this manner, the SDA method eliminated the need for complicated temperature control, which was required in PCR.

### Disclosure of the Invention

An object of the present invention is to provide a method for selectively and efficiently synthesizing a relatively long sense or antisense strand.

The present inventors have conducted concentrated studies, and as a result, they found that a single-stranded nucleic acid could be selectively and efficiently synthesized. This involves the use of a method for amplifying DNA utilizing the LAMP method and a "restriction enzyme which is capable of forming a fragment having an overhanging 3' terminus and cleaving so as to make base sequences of the single-stranded regions of the fragments after the cleavage different from each other." This led to the completion of the present invention. More specifically, the present invention comprises the following.
(1) A method for synthesizing a single-stranded nucleic acid comprising the following steps of:
   1) cleaving, with a restriction enzyme, double-stranded DNA having a restriction enzyme recognition sequence at a portion closer to the 5' side than the target sequence in such a manner that
      a) a fragment having an overhanging 3' terminus is formed, and
      b) base sequences of the single-stranded regions of the fragments after the cleavage differ from each other;
   2) to a single-stranded region of the DNA fragment that was cleaved with the restriction enzyme, annealing a primer having a base sequence complementary to the region on at least its 3' terminus; and
   3) synthesizing a nucleic acid by a strand displacement-type polymerase starting from the 3' terminus of the primer.
(2) The method according to (1) above, wherein the double-stranded DNA having the restriction enzyme recognition sequence is provided by a method comprising the following steps of:
   1) cloning the DNA to be amplified using a vector having the restriction enzyme recognition sequence in the cloning site or adjacent to the cloning site; and
   2) amplifying the region containing the restriction enzyme recognition sequence and the DNA to be amplified by the DNA amplification method using a primer that anneals on the restriction enzyme recognition sequence or the 3' side thereof.
(3) The method according to (2) above, wherein the DNA amplification method is the LAMP method.
(4) The method according to (1) above, wherein the double-stranded DNA having the restriction enzyme recognition sequence is provided by the DNA amplification method using a primer containing the restriction enzyme recognition sequence.
(5) The method according to (4) above, wherein the DNA amplification method is the LAMP method using the primers described in the following A) and B):
   when a first arbitrary sequence F1c and a second arbitrary sequence F2c are selected in that order from the 3' terminus of the target sequence on the first DNA strand of the double-stranded DNA toward the 3' terminus on the DNA strand, and a third arbitrary sequence R1 and a fourth arbitrary sequence R2 are selected in that order from the 5' terminus of the target sequence toward the 5' terminus on the DNA strand,
   A) a primer containing sequence F2, which is complementary to F2c, and the same sequence as F1c in that order from the 3' side toward the 5' side or a primer containing sequence F2, which is complementary to F2c, the restriction enzyme recognition sequence, and the same sequence as F1c in that order from the 3' side toward the 5' side; and
   B) a primer containing the same sequence as R2, the restriction enzyme recognition sequence, and sequence R1c, which is complementary to R1, in that order from the 3' side toward the 5' side.
(6) The method according to (5) above, wherein the step of synthesizing DNA comprises the use of an outer primer that anneals to the portion closer to the 3' side than the inner primer.
(7) The method according to any one of (1) to (6) above, wherein the single-stranded region at the cleavage site created by the restriction enzyme comprises at least 5 bases.
(8) The method according to any one of (1) to (6) above, wherein the single-stranded region at the cleavage site created by the restriction enzyme comprises at least 7 bases.
(9) The method according to any one of (1) to (8) above,
   wherein the primer is bound to or modified to be bindable to a detectable label substance or solid phase.
(10) An inner primer pair comprising the following A) and B):
   when a first arbitrary sequence F1c and a second arbitrary sequence F2c are selected in that order from the 3' terminus of the target sequence on the first DNA strand of the double-stranded DNA toward the 3' terminus on the DNA strand and a third arbitrary sequence R1 and a fourth arbitrary sequence R2 are selected in that order from the 5' terminus of the target sequence toward the 5' terminus on the DNA strand,
   A) a primer containing sequence F2, which is complementary to F2c, and the same sequence as F1c in that order from the 3' side toward the 5' side, or a primer containing sequence F2, which is complementary to F2c, a recognition sequence of the following restriction enzyme, and the same sequences as F1c in that order from the 3' side toward the 5' side, wherein the restriction enzyme is capable of
      a) forming a fragment having an overhanging 3' terminus, and
      b) cleaving so as to make base sequences of the single-stranded regions of the fragments after the cleavage different from each other, and
   B) a primer containing the same sequence as R2, a recognition sequence of the restriction enzyme, and sequence R1c, which is complementary to R1, in that order from the 3' side toward the 5' side.
(11) A vector for synthesizing a single-stranded nucleic acid comprising the following base sequence in the cloning site or adjacent to the cloning site, wherein the base sequence is cleaved with a restriction enzyme in such a manner that
   a) a fragment with an overhanging 3' terminus is formed, and
   b) base sequences of the single-stranded regions of the fragments after the cleavage differ from each other.
(12) A reagent kit for synthesizing a single-stranded nucleic acid comprising at least the following reagents:
   1) a restriction enzyme capable of:
      a) forming a fragment having an overhanging 3' terminus, and
      b) cleaving so as to make the base sequences of fragments different from each other,
   2) a primer capable of annealing to the single-stranded region of the DNA fragment cleaved with the restriction enzyme;
   3) a strand displacement-type polymerase; and
   4) the inner primer according to (10) above or the vector for synthesizing a single-stranded nucleic acid according to (11) above.

In the method for synthesizing a single-stranded nucleic acid according to the present invention, the term "single-stranded nucleic acid" refers to DNA which consists of either one of the sense or antisense strands of the double-stranded DNA, i.e., single-stranded DNA. The term "annealing" refers to the formation by a nucleic acid of a double-stranded structure by base pairing in accordance with the law of Watson-Crick. Accordingly, if a single-stranded nucleic acid forms such base pairing in a molecule, it is "annealing." In the present invention, "annealing" and "hybridization" are synonymous with each other in that a nucleic acid has a double-stranded structure formed by base pairing.

In the present invention, the term "target sequence (or region)" refers to a base sequence (or region) which should be synthesized as a single-stranded nucleic acid. Of the template double-stranded DNA, the strand containing the target sequence refers to the first strand and the strand complementary thereto refers to the second strand.

Techniques according to the LAMP method are applied in the present invention. The LAMP method is a method for amplifying a nucleic acid, which was developed by the present inventors. In this method, an inner primer pair or two or four specific primers prepared by adding an outer primer pair to the inner primer pair, a strand displacement-type DNA polymerase, and a substrate nucleotide are used to rapidly and cost-effectively amplify DNA or RNA under isothermal conditions (at approximately 65°C). The LAMP method is described in detail in, for example, Nagamine et al., Clinical Chemistry (2001), Vol. 47, No. 9, 1742-1743, Notomi et al., Nucleic Acids Research (2000), Vol. 28, e63, and International Application (e.g., WO 00/28082, WO 01/34838, and WO 01/34790). These descriptions are incorporated herein by reference.

The present invention utilizes a restriction enzyme, which is capable of a) forming a fragment having an overhanging 3' terminus, and b) cleaving so as to make base sequences of the single-stranded regions of the fragments after the cleavage different from each other. After the cleavage, most restriction enzymes form fragments having overhanging 5' terminuses or fragments having blunt terminuses. However, a minor number of restriction enzymes that form fragments having the overhanging 3' terminuses after the cleavage are known. A primer is annealed to the single-stranded region of the DNA fragment which was cleaved using such enzymes, and a nucleic acid can be synthesized by a polymerase. In order to conduct the synthesis reaction of a nucleic acid by annealing a primer to the overhanging 3' terminal region, the single-stranded region should comprise at least 5 bases, and preferably at least 7 bases when the specificity of the base sequence and the binding strength are taken into consideration.

Some restriction enzymes have cleavage sites in an arbitrary sequence sandwiched between recognition sites or adjacent to a recognition site. For example, Bg1I cleaves between the base 7 and the base 8 in the sequence GCCNNNNNGGC. The fragment cleaved with such a restriction enzyme produces an asymmetric fragment having different base sequences in the cleavage sites. Specifically, when the sequence GCCAAAAAGGC is cleaved with Bg1I, the base sequences of the cleavage site are GCCAAAA and GCCTTTT. In such asymmetric fragments, a primer (probe) having a base sequence complementary to one of them cannot bind to the other.

One example of a restriction enzyme having both characteristics mentioned above (hereinafter it is referred to as "the restriction enzyme of the present invention") is TspRI. TspRI recognizes the base sequence NNCA (C or G) TGNN and cleaves between an arbitrary base at the 3' terminus and a base adjacent thereto. Thus, the single-stranded region after the cleavage comprises 9 bases.

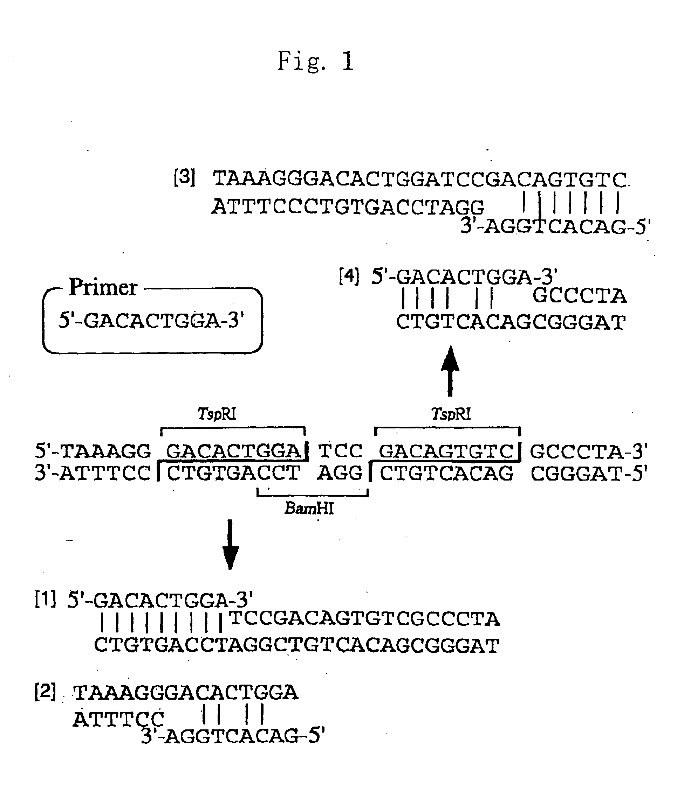

The principle of the method for synthesizing a single-stranded nucleic acid according to the present invention is described using TspRI as an example. As mentioned above, TspRI is a restriction enzyme that has an overhanging terminus of 9 bases at the cleavage surface on the 3' side. Further, 2 bases (NN) at the both terminuses of the recognition sequence NNCA (C/G) TGNN may be any of A, T, G, or C, and an asymmetric recognition sequence can be formed. Utilization thereof enables the strand-specific DNA synthesis. For example, a sequence having TspRI sites at the both ends of the BamHI cloning site is prepared. Digestion thereof with TspRI can yield four 3'-overhanging terminuses having different base sequences. Subsequently, a primer (GACACTGGA) is prepared from a sequence which recognizes one of the four terminuses (Fig. 1 [1]). This primer is not a sequence that completely matches [2], [3], or [4], and thus, cannot be annealed. Accordingly, when the primer is annealed to [1] and causes the extension reaction, the synthesis reaction takes place using only the same strand as a template.

The techniques of the LAMP method mentioned above (Nucleic Acids Research (2000), Vol. 28, e63) can be applied to the above synthesis reaction. For example,
1) DNA to be amplified is cloned using a vector having the restriction enzyme recognition sequence in a cloning site or adjacent to the cloning site;
2) subsequently, a primer for LAMP to be annealed on the restriction enzyme recognition sequence or the 3' side therefrom is designed; and
3) the primer and the strand displacement-type polymerase are used to amplify by the LAMP method the region containing the restriction enzyme recognition sequence and DNA to be amplified.

Processes for synthesizing single-stranded DNA from double-stranded DNA having the amplified restriction enzyme recognition sequence are as described above. Since the LAMP method can provide a large amount of DNA under isothermal conditions in a short period of time, more strand-specific single-stranded DNA can be efficiently obtained by combining the LAMP method with the above synthesis method.

The primer for LAMP refers to a specific primer, which is used in the LAMP method, and can be easily prepared based on, for example, the aforementioned Nagamine et al., "Clinical Chemistry (2001)."

A primer, which is used for synthesizing the single-stranded DNA, is not particularly limited as long as it complementarily binds to the overhanging single-stranded portion at the 3' terminus existing on the second DNA strand, which was cleaved with a restriction enzyme. The length is not necessarily completely consistent with that of the single-stranded region. It may be shorter than the single-stranded region on the 5' side or the 3' side, or longer on the 5' side, as far as the specificity of complementary binding is not deteriorated.

A primer may be bound to, or modified to be bindable to, a detectable label substance or solid phase. When labeling the primer for synthesizing single-stranded DNA, known substances and methods for labeling can be employed. Examples of label substances include radioactive substances, fluorescent substances, biotins, and enzymes. These label substances can be added to a primer in accordance with a known method, or a previously labeled nucleotide can be incorporated at the time of chemical synthesis of a primer to prepare a label primer. A suitable functional group may be introduced in the primer so as to be bindable to the aforementioned label substances or latex particles, magnetic particles, or the inner wall of a reaction vessel.

The label site of the primer has to be selected in such a manner that annealing to a complementary strand or a subsequent extension reaction is not inhibited. Thus, for example, a label at the 3' terminus is not preferable. Depending on their molecular weight, label substances can be bound through a base sequence as a linker on the 5' side to prevent steric hindrance from occurring.

In the method for synthesizing a single-stranded nucleic acid according to the present invention, the recognition sequence can be inserted into the double-stranded DNA that does not contain a restriction enzyme recognition sequence of the present invention by conventional methods for gene amplification such as PCR and LCR. For example, the restriction enzyme recognition sequence of the present invention is incorporated into a primer to be used, and the target sequence or a sequence complementary thereto is amplified. Thus, the restriction enzyme recognition sequence of interest can be inserted on the 3' side of the target sequence.

As mentioned above, a vector, which comprises the restriction enzyme recognition sequence of the present invention incorporated in the cloning site of the cloning vector or adjacent to the cloning site, may be used.

According to a preferred embodiment of the present invention, the restriction enzyme recognition sequence can be introduced into double-stranded DNA that does not contain a restriction enzyme recognition sequence of the present invention by the LAMP method. Specifically, a large amount of template DNA strands for synthesizing a single-stranded nucleic acid having a restriction enzyme recognition sequence can be rapidly prepared by performing LAMP amplification using a primer comprising the restriction enzyme recognition sequence inserted therein.

The restriction enzyme recognition sequences should be inserted on the 5' side of the target sequence on the first strand. Particularly, it is more preferable if the sequences are inserted into both the 3' side and the 5' side. More specifically, the restriction enzyme recognition sequence is always necessary for the primer which is annealed to the overhanging 3' portion on the second strand, and it is more preferable if the sequences are contained in both the primers. This is because the presence of restriction sites on both sides allows cleavage of both sides of the target region by one type of enzyme and can decrease the necessary number of enzymes. It should be noted that the base sequences of the single-stranded regions of the fragments, which are obtained by cleaving the extension product from each primer with the restriction enzyme, are preferably different from each other.

In the present invention, the above two types of primers are referred to as "inner primers."

More specifically, the method for introducing a restriction enzyme recognition sequence into double-stranded DNA by the LAMP method comprises the following steps of:
1) selecting a first arbitrary sequence F1c and a second arbitrary sequence F2c in that order from the 3' terminus of the target sequence on the first DNA strand of the double-stranded DNA toward the 3' terminus on the DNA strand and selecting a third arbitrary sequence R1 and a fourth arbitrary sequence R2 in that order from the 5' terminus of the target sequence toward the 5' terminus on the DNA strand;
2) preparing the following inner primers:
   a) a primer containing sequence F2, which is complementary to F2c, and the same sequence as F1c in that order from the 3' side toward the 5' side, or a primer containing sequence F2, which is complementary to F2c, the restriction enzyme recognition sequence, and the same sequences as sequence F1c in that order from the 3' side toward the 5' side; and
   b) a primer containing the same sequence as R2, the restriction enzyme recognition sequence, and sequence R1c, which is complementary to R1, in that order from the 3' side toward the 5' side; and
3) synthesizing DNA using each strand of the double-stranded DNA as a template and using the primer and the strand displacement-type polymerase.

In the aforementioned inner primers, F2 may overlap with the restriction enzyme recognition sequence, and the restriction enzyme recognition sequence may overlap with F1c, or several base sequences may be inserted therebetween. Similarly, R2 may overlap with the restriction enzyme recognition sequence, and the restriction enzyme recognition sequence may overlap with R1c, or several base sequences may be inserted therebetween. Preferably, distance between F2/R2 and the restriction enzyme recognition sequence or distance between the restriction enzyme recognition sequence and F1c/R1c is 1 to 20 bases, and more preferably 1 to 10 bases.

The first annealing of the inner primer to double-stranded DNA can be carried out by dissociating a part or the entire double-stranded DNA by conventional methods such as by heat or alkali. Thereafter, DNA synthesis is repeated in the presence of a strand displacement-type DNA polymerase and a substrate nucleotide under isothermal conditions of approximately 55°C to 70°C.

In the aforementioned LAMP method, two types of primers that differ from the inner primers can be further used. These primers anneals to the outer side, i.e., closer to the 3' side, than the inner primers on the template DNA, and they are referred to as "outer primers" in the present invention. The outer primers can be prepared in the following manner.

On the first DNA strand, an arbitrary sequence F3c, which is located closer to the 3' side than F2c, and an arbitrary sequence R3, which is located closer to the 5' side than R2, are selected, and
a) a primer containing sequence F3, which is complementary to F3c, and
b) a primer containing a sequence that is identical to R3, are respectively prepared as the outer primers.

The DNA synthesis from the outer primer should be initiated after the DNA synthesis from the inner primer. Examples of methods for realizing this include: a method in which the concentration of the inner primer is set higher than that of the outer primer (e.g., 2 to 50 times, preferably 4 to 25 times) (a); and a method in which the melting temperature (Tm) of the inner primer is set higher than that of the outer primer (b). In addition, conditions for carrying out the LAMP method can be suitably determined with reference to the literature or patent mentioned above.

The LAMP method can be more efficiently carried out with the use of a primer capable of specifically annealing to the loop portion of the amplification product having a hairpin structure formed by amplification. In the present invention, the loop-specific primer is referred to as a "loop primer." If the restriction enzyme recognition sequence of the present invention is inserted into one of the 3' terminuses of the loop primer, the loop primer per se can function as a primer for synthesizing a single-stranded nucleic acid.

The present invention also provides a reagent kit for synthesizing a single-stranded nucleic acid for carrying out the method according to the present invention. This kit can be used, for example, as a reagent kit for synthesizing a single-stranded nucleic acid for the hybridization assay of a particular sequence.

The aforementioned kit comprises at least the restriction enzyme according to the present invention, a primer capable of annealing to the single-stranded region of the DNA fragment cleaved with the restriction enzyme, a strand displacement-type polymerase, and the inner primer according to the present invention. The kit may further comprise the outer primer and/or the loop primer according to the present invention.

The kit may further comprise, in addition to the aforementioned reagent, other reagents necessary for carrying out the synthesis method of the present invention, for example, a substrate nucleotide, a buffer, or a melting temperature regulator.

### Brief Description of the Drawings

Fig. 1 shows the principle for synthesizing the single-stranded nucleic acid using TspRI.
Fig. 2 is a photograph showing the result of the hybridization assay on the extension product in Example 1 (A: sense oligo, B: antisense oligo).
Fig. 3 is a photograph showing the result of electrophoresis on the LAMP reaction product in Example 2 and the TspRI-treated LAMP reaction product (M: size marker, 1: LAMP product, 2: TspRI-treated LAMP product).
Fig. 4 is a photograph showing the result of detecting the primer extension product (DIG-labeled) in Example 2 (1: negative control, 2: primer extension product).
Fig. 5 is a photograph showing the result of dot blot hybridization of the primer extension product in Example 2.
Fig. 6 is a photograph showing the result of detecting the primer extension product (DIG-labeled) in Example 3 (1: Klenow(-), 2: Klenow(+), 3: Bst(-) 60°C, 4: Bst(+) 60°C, 5: Bst(-) 65°C, 6: Bst(+) 65°C).
Fig. 7 is a photograph showing the result of electrophoresis on the lambda exonuclease-treated primer extension product in Example 4 (M: size marker, 1: λ(-), 2: λ(+)).
Fig. 8 is a photograph showing the result of detecting the primer extension product (DIG-labeled) in Example 5.
Fig. 9 is a photograph showing the result of dot blot hybridization of the primer extension product in Example 5.
Fig. 10 is a photograph showing the result of electrophoresis on the LAMP reaction product in Example 6 and the TspRI-treated LAMP reaction product (M: size marker, 1: LAMP product, 2: TspRI-treated LAMP product).
Fig. 11 is a photograph showing the result of dot blot hybridization of the primer extension product in Example 6.

This specification includes part or all of the contents as disclosed in the description of Japanese Patent Application No. 2000-328219, which is a priority document of the present application.

### Preferred Embodiments of the Present Invention

### Example 1:

### 1. Preparation of vector

PCR was carried out using the following primers, and the pBluescript II vector (Genbank DB No. X52324) as a template. T7TspRI (Forward): 5'-GACAGTGTCGCCCTATAGTGAGTCGTATTA-3' (SEQ ID NO: 1) T3TspRI (Reverse): 5'-GGATCCAGTGTCCCTTTAGTGAGGGTTAAT-3' (SEQ ID NO: 2)

To the 40 µl of reaction system were added 1 x buffer (16 mM (NH₄)₂SO₄, 50 mM Tris-HCl pH 9.2, 1.75 mM MgCl₂, 0.001% (w/v) gelatin), 0.2 mM dNTPs, 1.4 U Taq DNA polymerase, Pwo DNA polymerase (EXpand Long Template PCR System, Roche), and 0.5 mM specific primer pair mentioned above. PCR was repeated fifteen times under conditions of at 94°C for 20 seconds, at 62°C for 30 seconds, and at 68°C at 120 seconds.

The amplification product was blunt-ended using the T4 DNA polymerase, and ligation was carried out using the T4 DNA ligase. The ligation product was introduced into *Escherichia coli* DH5α, and a vector of interest was obtained from the resulting transformant (designated as "pBSTspRI").

A multiple cloning site from SacI to Kpnl (underlined portion) in the pBluescript II vector has been deleted from the vector obtained just above. Instead, the vector has the TspRI-BamHI-TspRI site, and can insert the foreign gene into the BamHI site.

### (A) pBluescript II vector :

5'-GAGCGCAACGCAATTAATGTGAGTTAGCTCACTCATTAGGCACCCCAGGCTTTACAC
TTTATGCTTCCGGCTCGTATGTTGTGTGGAATTGTGAGCGGATAACAATTTCACACAG
GAAACAGCTATGACCATGATTACGCCAAGCGCGCAATTAACCCTCACTAAAGGGAAC
AAAAGCTGGAGCTCCACCGCGGTGGCGGCCGCTCTAGAACTAGTGGATCCCCCGGG
CTGCAGGAATTCGATATCAAGCTTATCGATACCGTCGACCTCGAGGGGGGGCCCGGT
ACCCAATTCGCCCTATAGTGAGTCGTATTACGCGCGCTCACTGGCCGTCGTTTTACAA
CGTCGTGACTGGGAAAACCCTGGCGTTACCCAACTTAATCGCCTTGCAGCACATCCC
CCTTTCGCCAGCTGGCGTAATAGCGAAGAGGCCCGCACCG-3' (SEQ ID NO: 10)

The underlined sequence (SEQ ID NO: 11) indicates a multiple cloning site.

### (B) Sequence having the TspRI-BamHI-TspRI site:

5'-GGGACACTGGATCCGACAGTGTCGCCC-3'(SEQ ID NO: 3)

### 2. Cloning of DNA

pUC19 vector (GenBank DB No.: L09137) was digested with Sau3AI, and 109 bp and 82 bp DNA fragments were isolated. These fragments were inserted into the BamHI site of the vector and cloned.
109 bp Sau3AI fragment:
5'-GATCCTTTTAAATTAAAAATGAAGTTTTAAATCAATCTAAAGTATATATGAGTAAACTT GGTCTGACAGTTACCAATGCTTAATCAGTGAGGCACCTATCTCAGCGATC-3' (SEQ ID NO: 12)
82 bp Sau3AI fragment:
5'-GATCTTTTCTACGGGGTCTGACGCTCAGTGGAACGAAAACTCACGTTAAGGGATTTT GGTCATGAGATTATCAAAAAGGATC-3' (SEQ ID NO: 13)

### 3. LAMP reaction

The target region was amplified by the LAMP reaction (see Nucleic Acids Research (2000), Vol. 28, e63) using the vector comprising the Sau3AI fragment incorporated therein as a template. The composition of the reaction solution used is shown in Table 1, and the sequence and the concentration of the primer are shown below.

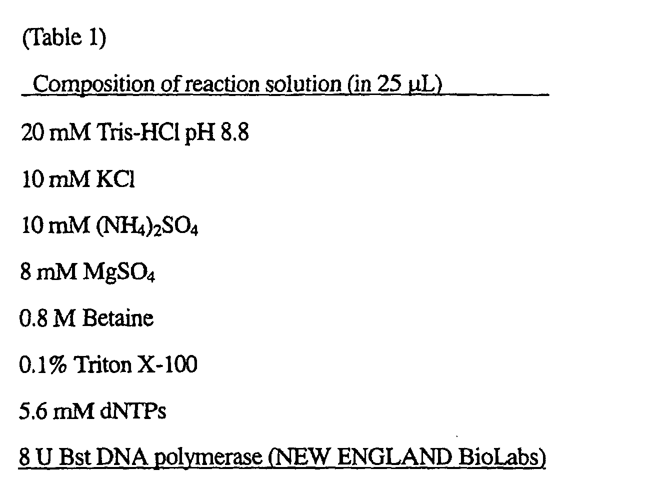

### Primer:

### Inner primer (1600 nM)

Forward:
5'-TCCAGTGTCCCTTTAGTGAGGGTTAATTTCACACAGGAAACAGCTATGACCA-3' (SEQ ID NO: 4)

Reverse:
5'-GACAGTGTCGCCCTATAGTGAGTCGTATTACCAGTCACGACGTTGTAAAACGA-3' (SEQ ID NO: 5)

### Outer primer (400 nM)

Forward: 5'-GTAACGCCAGGGTTTTCC-3' (SEQ ID NO: 6)

Reverse: 5'-GAATTGTGAGCGGATAACAAT-3' (SEQ ID NO: 7)

### Loop primer (800 nM)

Forward: 5'-GCGCGCTTGGCGTAATCA-3' (SEQ ID NO: 8)

Reverse: 5'-GCGCGCTCACTGGCCG-3' (SEQ ID NO: 9)

Target DNA, which is not heat-denatured, was prepared, and the reaction solution was allowed to react at 65°C. The product was eluted to 200 µl of Tris-HCl (pH 8.0) using the PCR Purification Kit (QIAGEN).

### 3. Digestion with TspRI

The LAMP product (25 µl) was allowed to react in 50 U of TspRI (NEW ENGLAND BioLabs) at 65°C for 90 minutes. After the reaction, the product was eluted to 100 µl of Tris-HCl (pH 8.0) using the PCR Purification Kit (QIAGEN).

### 4. Primer extension reaction

A primer labeled with 5'-digoxigenin (DIG) (BSTspRI: 5'-GACACTGGA-3') was added to the TspRI fragment, and the extension reaction using DNA polymerase Klenow fragment, 3' → 5'exo- (NEW ENGLAND BioLabs) was carried out. The composition of the reaction solution used is shown in Table 2.

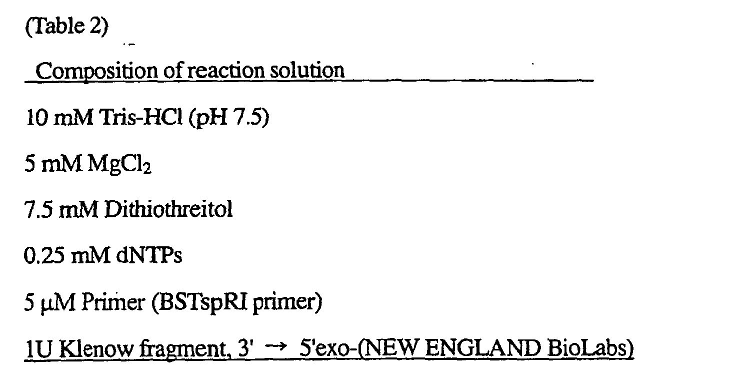

In this case, the primer BSTspRI can be annealed to only one cohesive terminus of the TspRI-digested fragment, and thus, there should be a single DNA strand obtained by the extension reaction.

### 5. Dot blot hybridization

A sense oligo (109A oligo) or antisense oligo (109B oligo), which was designed from the base sequence of the 109 bp Sau3AI DNA fragment, was blotted on a nylon membrane filter (BiodyneB, Pall). Sequences are shown below.
109A oligo: 5'-GTCTGACAGTTACCAATGCTTAATCAGTGAGGCACCTATCTCAGC-3'
(SEQ ID NO: 15)
109B oligo: 5'-GCTGAGATAGGTGCCTCACTGATTAAGCATTGGTAACTGTCAGAC-3'
(SEQ ID NO: 16)

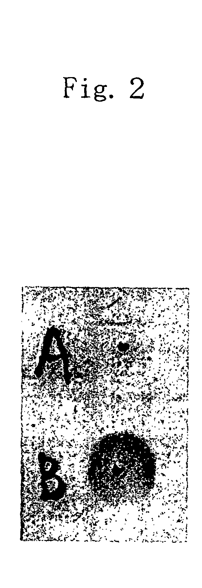

This filter was subjected to hybridization using the extension product as a probe. Hybridization was carried out using PerfectHyb buffer (TOYOBO) at 60°C overnight. Signals were detected using the DIG Nucleic Acid Detection Kit (Roche). As a result, a signal was detected only on the portion where the antisense oligo had been blotted (Fig. 2). This indicates that one strand-specific single-strand DNA was obtained.

### Example 2:

The aforementioned 82 bp cloning product was used to conduct the experiment in order to confirm whether or not a similar effect could be attained with another template.

The 82 bp cloning vector that was prepared in Example 1 was used as a template to perform the LAMP reaction under the same conditions as above. In this LAMP reaction, the following inner primers and the same outer primers as used in Example 1 (the same concentration as in Example 1) were used instead of the loop primer.

### Inner primer (1600 nM)

Forward: 5'-GTGTCCCTTTAGTGAGGGTTAATTTCACACAGGAAACAGCTATG-3' (SEQ ID NO: 17) Reverse: 5'-TGTCGCCCTATAGTGAGTCGTATTACCAGTCACGACGTTGTAAA-3' (SEQ ID NO: 18)

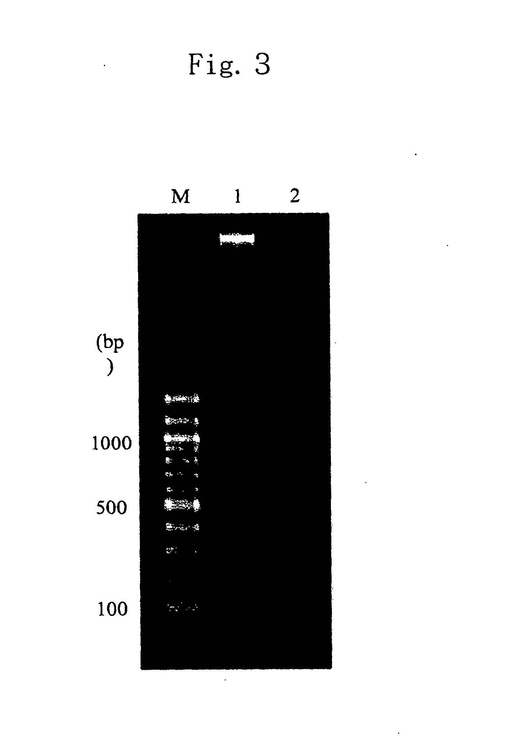

The LAMP reaction product was treated with TspRI and confirmed by 2% agarose gel electrophoresis (Fig. 3).

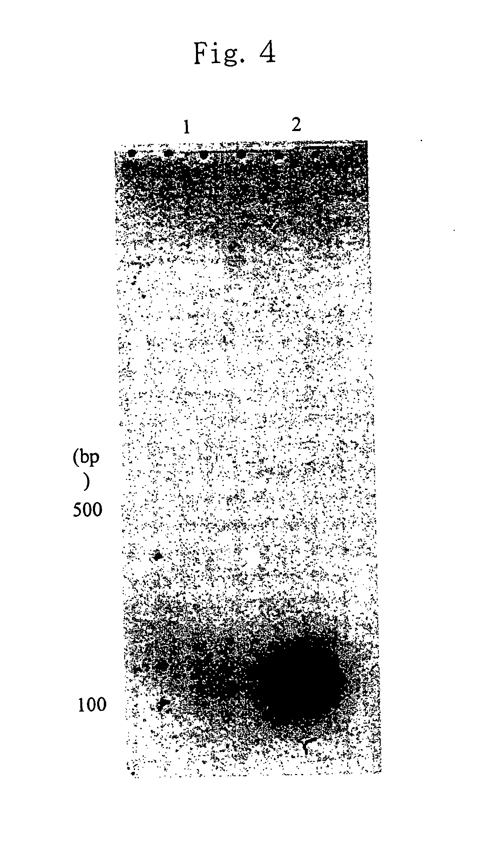

After the confirmation of the complete digestion with TspRI by electrophoresis, primer extension was carried out in the same manner as in Example 1. In order to confirm the implementation of primer extension, the product was electrophoresed in 2% agarose gel and transferred to the nylon membrane (BiodyneB, Pall). Whether or not this membrane contained the label substance in the electrophoresis product was detected using the DIG Nucleic Acid Detection Kit (Roche) (Fig. 4). As a result, it was confirmed that the extension product from the DIG-labeled primer was contained in the TspRI product.

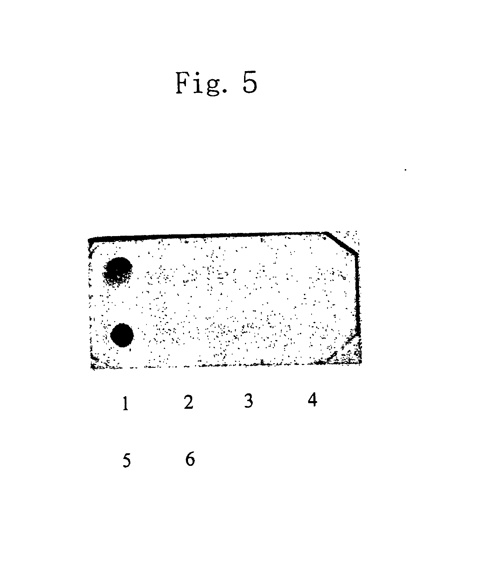

Since the product was confirmed as being labeled, dot blot hybridization was carried out using this product as a probe (Fig. 5). The blotted DNAs are as follows.
1: 82 bp antisense oligo DNA (SEQ ID NO: 14)
2: 82 bp sense oligo DNA (SEQ ID NO: 13)
3: 109A oligo
4: 109B oligo
5: heat-denatured product of 82 bp LAMP product
6: heat-denatured product of unrelated LAMP product (λ DNA amplification product)

As a result, signals were found only in 1 and 5. This indicates that, even with a different template, only the strand-specific DNA was synthesized as with Example 1. Since a signal was also found in the heat-denatured product of the LAMP product, it was found that the LAMP product per se could be a target of the probe. Due to its structure, i.e., the presence of the repeated sequence, the LAMP product was considered to be very poor in the hybridization efficiency. In this study, since hybridization was carried out with the LAMP product, the possibility of the use of the LAMP product by spotting on the substrate of the DNA chip was suggested.

A primer, which was DIG-labeled at its 5' terminus, was used in this study. If an amino linker or a biotin-labeled primer is used, the labeled DNA strand can be isolated.

### Example 3: Extension reaction using Bst DNA polymerase

The Bst DNA polymerase, i.e., a strand displacement-type DNA synthetase, was used to determine whether or not the primer extension reaction could be carried out. The composition of the reaction solution used is shown in Table 3.

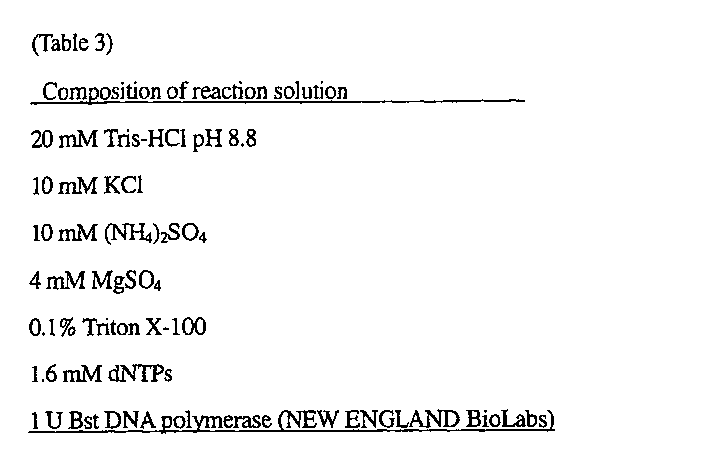

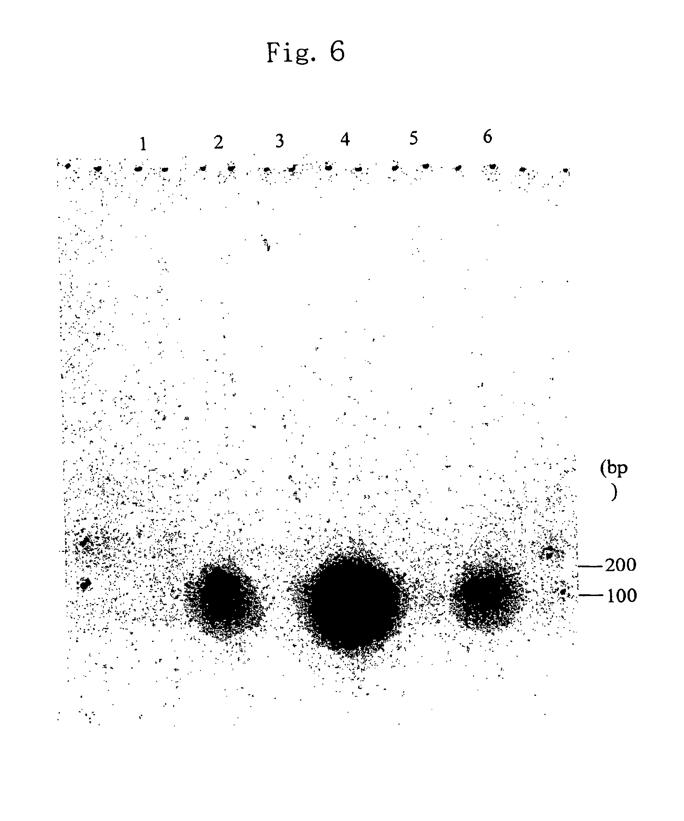

After the reaction at 60°C or 65°C for 1 hour, the product was electrophoresed in 2% agarose gel and transferred to a nylon membrane (BiodyneB, Pall) in order to confirm the implementation of primer extension. Whether or not this membrane contained the label substance in the electrophoresis product was detected using the DIG Nucleic Acid Detection Kit (Roche) (Fig. 6). As a result, the occurrence of extension from the DIG-labeled primer with the use of the Bst DNA polymerase was confirmed.

### Example 4: Lambda exonuclease treatment of primer extension product

After the primer extension, free single-stranded DNA and double-stranded DNA are generated. The strand extended from the primer of the double-stranded DNA does not have a phosphoric acid group at its 5' terminus. Thus, if the strand is subjected to lambda exonuclease treatment, DNA decomposition starts from the 5' side of the template DNA strand, although the extended strand is not decomposed. This results in the presence of the DNA strand only on one side.

The aforementioned primer extension product (109 bp primer extension product) was subjected to lambda exonuclease treatment (reaction at 37°C for 1 hour). The composition of the reaction solution used is shown in Table 4.

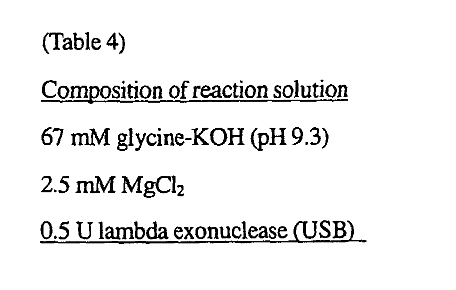

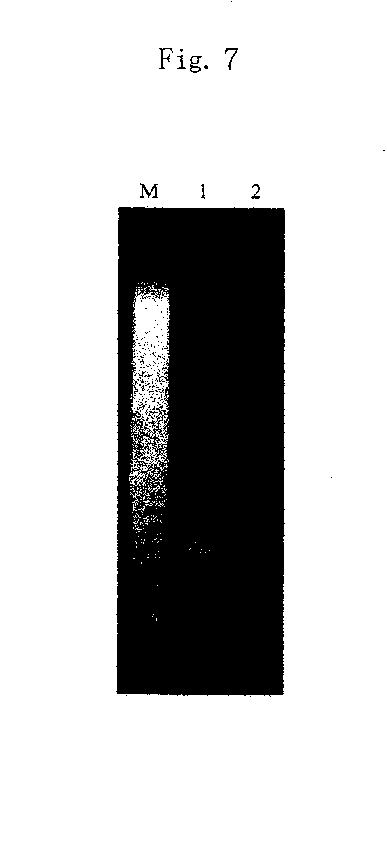

After the reaction at 37°C for 1 hour, electrophoresis was carried out in 4% agarose gel. The primer extension product was subjected to lambda exonuclease treatment and electrophoresis, and as a result, a band was observed to have been shifted to around 50 bp (Lane 2 in Fig. 7). The rate of migration of the single-stranded DNA is known to have increased, and this was considered to be the product of interest. Accordingly, it was confirmed that a larger amount of single-stranded DNA could be obtained by treating the primer extension product with lambda exonuclease.

### Example 5: Bst DNA polymerase extension reaction using TspRI buffer

### 1. The reaction with the Bst DNA polymerase was carried out using TspRI buffer.

The LAMP product used as a template was the same as in Example 2. In this case, dNTPs having final concentrations of 0, 0.25, 0.5, 1.0, and 2.0 mM were used. The composition of the reaction solution used is shown in Table 5.

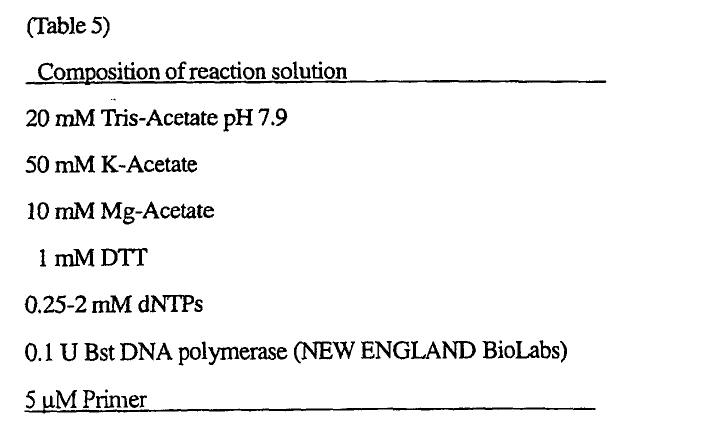

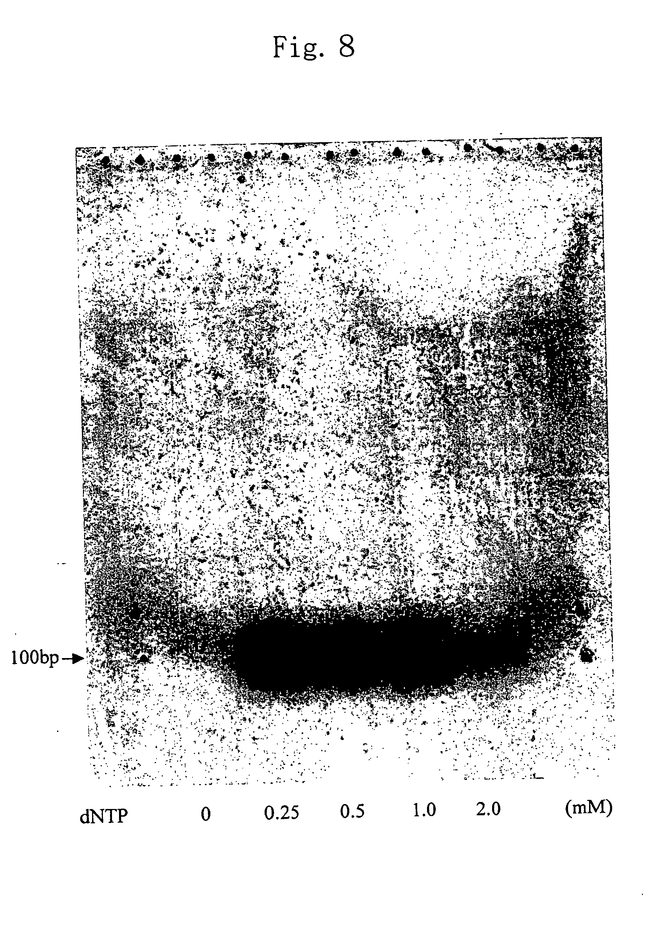

After the reaction at 37°C for 1 hour, the reaction product was electrophoresed in 2% agarose gel and transferred to a nylon membrane (BiodyneB, Pall) in order to confirm the implementation of primer extension. Whether or not this membrane contained the label substance in the electrophoresis product was detected using the DIG Nucleic Acid Detection Kit (Roche) (Fig. 8). As a result, the occurrence of extension from the primer by the Bst DNA polymerase with the use of TspRI buffer was confirmed.

This indicates that use of this method can decrease a necessary cost by sharing a buffer. 2. Since the occurrence of extension with the use of the TspRI buffer was confirmed, whether or not the reaction subsequent to the LAMP could be simultaneously carried out was then examined. The composition of the reaction solution used is shown in Table 6.

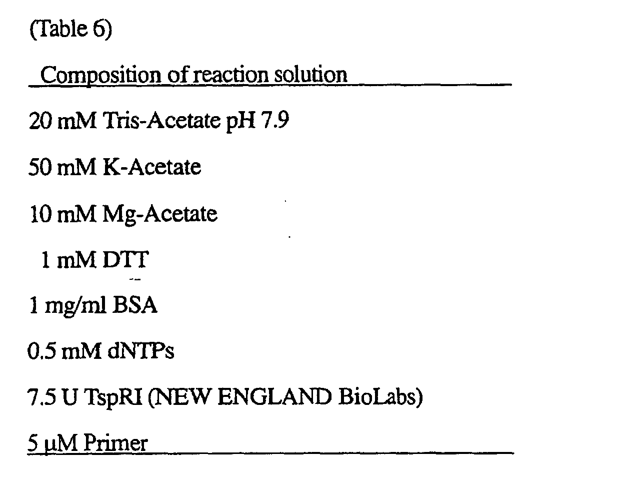

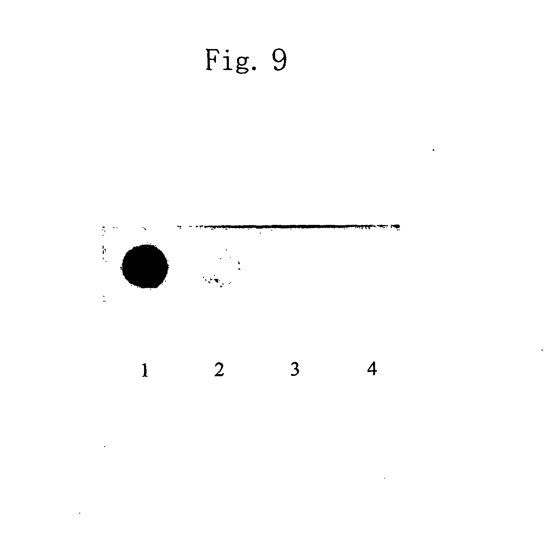

The reaction solution (1/50 amount) after the LAMP reaction was added to the reaction system and allowed to react at 37°C for 1 hour. The amount of the Bst DNA polymerase in the reaction solution was 0.16 U. It was confirmed in the Examples that 0.1 U of enzyme is sufficient to proceed the reaction. After the reaction, dot blot hybridization was carried out using the product as a probe (Fig. 9). The blotted DNAs were as follows.
1: 82 bp antisense oligo DNA
2: 82 bp sense oligo DNA
3: 109A oligo
4: 109B oligo

As a result, a potent signal was observed at position (1) where the 82 bp antisense oligo was blotted. A weak signal observed on the sense oligo side (2) was considered to be generated due to the portion that remained uncleaved in the reaction by TspRI. Thus, the production of specific amplification product was confirmed.

These results indicate that the use of this method can decrease cost as well as shorten necessary time.

### Example 6: Insertion of restriction enzyme recognition sequence using LAMP primer

1. In order to obtain a strand-specific single-strand from a template containing no TspRI site, the LAMP reaction was carried out using the following inner primers, etc. having the TspRI recognition sequence inserted therein and, as a template, 5 ng of pUC 19 plasmid (SEQ ID NO: 19).

### Inner primer (1600 nM: the underlined portion indicates the TspRI recognition sequence-inserted portion)

Forward:
5'-GACCAAGTTTACTCATATATACGACACTGGAGATCCTTTTAAATTAAAAATGAAG-3' (SEQ ID NO: 20)

Reverse:
5'-TGAGGCACCTATCTCAGCGATGACAGTGTCACGGGGAGTCAGGCAACTAT-3' (SEQ ID NO: 21)

### Outer primer (400 nM)

Forward: 5'-TCAAAAAGGATCTTCACCTA-3' (SEQ ID NO: 22) Reverse: 5'-GTATCGTAGTTATCTACACG-3' (SEQ ID NO: 23)

### Loop primer (800 nM)

Forward: 5'-ACTTTAGATTGATTTAAAA-3' (SEQ ID NO: 24) Reverse: 5'-TCTGTCTATTTCGTTCATCC-3' (SEQ ID NO: 25)

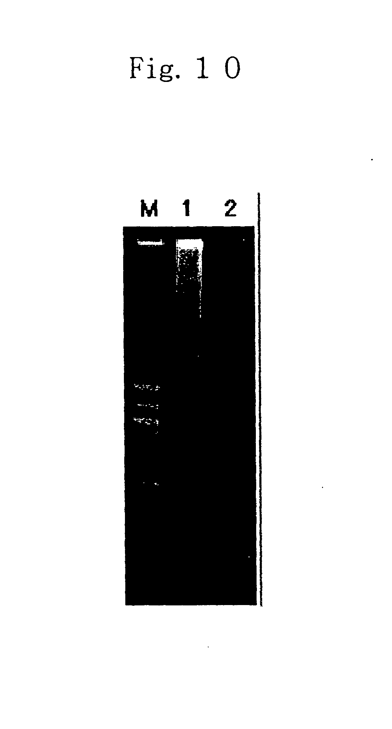

The composition of the reaction solution was the same as in Example 1, and the LAMP reaction was carried out at 62.8°C for 2 hours. Thereafter, the LAMP amplification product was digested with TspRI in the same manner as in Example 1. The LAMP product and the TspRI digest were electrophoresed in 2% agarose gel, and as a result, these were confirmed to be sizes of interest (Fig. 10).
2. Subsequently, DIG-labeling was carried out by primer extension and dot blot hybridization was carried out. The blotted DNAs were as follows.
1: 109A oligo
2: 109B oligo

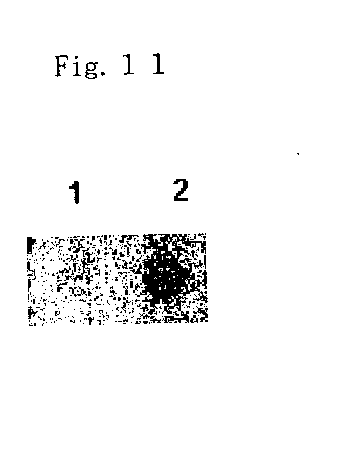

As a result, it was confirmed that a signal could be generated only at the spot of interest (Fig. 11).

This indicates that the restriction enzyme recognition sequence can be inserted into a template containing no TspRI site by the LAMP method, and, by using this as a template, a strand-specific single strand can be obtained.

All publications, patents, and patent applications cited herein are incorporated herein by reference in their entirety.

### Industrial Applicability

According to the present invention, either one of the sense or antisense strand of the double-stranded nucleic acid can be selectively synthesized. This single-stranded DNA can be used as a probe, etc. for hybridization assay. The present invention provides an efficient and cost-effective method for synthesizing single-stranded DNA.

### Free Text of Sequence Listing

SEQ ID NO: 1: T7TspRI primer (Forward)
SEQ ID NO: 2: T7TspRI primer (Reverse)
SEQ ID NO: 3: TspRI-BamHI-TspRI site
SEQ ID NO: 4: Inner primer (Forward)
SEQ ID NO: 5: Inner primer (Reverse)
SEQ ID NO: 6: Outer primer (Forward)
SEQ ID NO: 7: Outer primer (Reverse)
SEQ ID NO: 8: Loop primer (Forward)
SEQ ID NO: 9: Loop primer (Reverse)
SEQ ID NO: 10: pBluescript II
SEQ ID NO: 11: Multiple cloning site from pBluescript II
SEQ ID NO: 12: pUC19 109 bp Sau3AI fragment (sense)
SEQ ID NO: 13: pUC19 82 bp Sau3AI fragment (sense)
SEQ ID NO: 14: pUC19 82 bp Sau3AI fragment (antisense)
SEQ ID NO: 15: 109A oligo: Designed oligonucleotide (sense) based on pUC19 109 bp Sau3AI fragment)
SEQ ID NO: 16: 109B oligo: Designed oligonucleotide (antisense) based on pUC19 109 bp Sau3AI fragment)
SEQ ID NO: 17: Inner primer (Forward)
SEQ ID NO: 18: Inner primer (Reverse)
SEQ ID NO: 19: pUC19
SEQ ID NO: 20: Inner primer (Forward)
SEQ ID NO: 21: Inner primer (Reverse)
SEQ ID NO: 22: Outer primer (Forward)
SEQ ID NO: 23: Outer primer (Reverse)
SEQ ID NO: 24: Loop primer (Forward)
SEQ ID NO: 25: Loop primer (Reverse)

## Claims

1. A method for synthesizing a single-stranded nucleic acid comprising the following steps of:
1) cleaving, with a restriction enzyme, double-stranded DNA having a restriction enzyme recognition sequence at a portion closer to the 5' side than the target sequence in such a manner that
a) a fragment having an overhanging 3' terminus is formed, and
b) base sequences of the single-stranded regions of the fragments after the cleavage differ from each other;
2) to the single-stranded region of the DNA fragment that was cleaved with the restriction enzyme, annealing a primer having a base sequence complementary to the region on at least its 3' terminus; and
3) synthesizing a nucleic acid by a strand displacement-type polymerase starting from the 3' terminus of the primer.

2. The method according to claim 1, wherein the double-stranded DNA having the restriction enzyme recognition sequence is provided by a method comprising the following steps of:
1) cloning the DNA to be amplified using a vector having the restriction enzyme recognition sequence in the cloning site or adjacent to the cloning site; and
2) amplifying the region containing the restriction enzyme recognition sequence and the DNA to be amplified by the DNA amplification method using a primer that anneals on the restriction enzyme recognition sequence or the 3' side thereof.

3. The method according to claim 2, wherein the DNA amplification method is the LAMP method.

4. The method according to claim 1, wherein the double-stranded DNA having the restriction enzyme recognition sequence is provided by the DNA amplification method using a primer containing the restriction enzyme recognition sequence.

5. The method according to claim 4, wherein the DNA amplification method is the LAMP method using the primers described in the following A) and B):
when a first arbitrary sequence F1c and a second arbitrary sequence F2c are selected in that order from the 3' terminus of the target sequence on the first DNA strand of the double-stranded DNA toward the 3' terminus on the DNA strand, and a third arbitrary sequence R1 and a fourth arbitrary sequence R2 are selected in that order from the 5' terminus of the target sequence toward the 5' terminus on the DNA strand,
A) a primer containing sequence F2, which is complementary to F2c, and the same sequence as sequence F1c in that order from the 3' side toward the 5' side or a primer containing sequence F2, which is complementary to F2c, the restriction enzyme recognition sequence, and the same sequence as F1c in that order from the 3' side toward the 5' side; and
B) a primer containing the same sequence as R2, the restriction enzyme recognition sequence, and sequence R1c, which is complementary to R1, in that order from the 3' side toward the 5' side.

6. The method according to claim 5, wherein the step of synthesizing DNA comprises the use of an outer primer that anneals to the portion closer to the 3' side than the inner primer.

7. The method according to any one of claims 1 to 6, wherein the single-stranded region at the cleavage site created by the restriction enzyme comprises at least 5 bases.

8. The method according to any one of claims 1 to 6, wherein the single-stranded region at the cleavage site created by the restriction enzyme comprises at least 7 bases.

9. The method according to any one of claims 1 to 8, wherein the primer is bound to or modified to be bindable to a detectable label substance or solid phase.

10. An inner primer pair comprising the following A) and B):
when a first arbitrary sequence F1c and a second arbitrary sequence F2c are selected in that order from the 3' terminus of the target sequence on the first DNA strand of the double-stranded DNA toward the 3' terminus on the DNA strand and a third arbitrary sequence R1 and a fourth arbitrary sequence R2 are selected in that order from the 5' terminus of the target sequence toward the 5' terminus on the DNA strand,
A) a primer containing sequence F2, which is complementary to F2c, and the same sequence as F1c in that order from the 3' side toward the 5' side, or a primer containing sequence F2, which is complementary to F2c, a recognition sequence of the following restriction enzyme, and the same sequences as sequence F1c in that order from the 3' side toward the 5' side, wherein the restriction enzyme is capable of
a) forming a fragment having an overhanging 3' terminus, and
b) cleaving so as to make base sequences of the single-stranded regions of the fragments after the cleavage differ from each other, and
B) a primer containing the same sequence as R2, a recognition sequence of the restriction enzyme, and sequence R1c, which is complementary to R1, in that order from the 3' side toward the 5' side.

11. A vector for synthesizing a single-stranded nucleic acid comprising the following base sequence in the cloning site or adjacent to the cloning site, wherein the base sequence is cleaved with a restriction enzyme in such a manner that
a) a fragment with an overhanging 3' terminus is formed, and
b) base sequences of the single-stranded regions of the fragments after the cleavage differ from each other.

12. A reagent kit for synthesizing a single-stranded nucleic acid comprising at least the following reagents:
1) a restriction enzyme capable of:
a) forming a fragment having an overhanging 3' terminus, and
b) cleaving so as to make the base sequences of fragments different from each other,
2) a primer capable of annealing to the single-stranded region of the DNA fragment cleaved with the restriction enzyme;
3) a strand displacement-type polymerase; and
4) the inner primer according to claim 10 or the vector for synthesizing a single-stranded nucleic acid according to claim 11.


SEQUENCE LISTING

<110> Eiken Chemical Co., Ltd.

<120> Methods of synthesizing single strand nucleic acids

<130> WP-038

<150> JP2000-328219
<151> 2000-10-27

<160> 25

<170> PatentIn Ver. 2.1

<210> 1
<211> 30
<212> DNA
<213> Artificial Sequence

<220>
<223> Description of Artificial Sequence: T7TspRI primer(Forward)

<400> 1
gacagtgtcg ccctatagtg agtcgtatta                                    30

<210> 2
<211> 30
<212> DNA

<213> Artificial Sequence

<220>
<223> Description of Artificial Sequence:T7TspRI primer(Reverse)

<400> 2
ggatccagtg tccctttagt gagggttaat                                    30

<210> 3
<211> 27
<212> DNA
<213> Artificial Sequence

<220>
<223> Description of Artificial Sequence:TspRI-BamHI-TspRI site

<400> 3
gggacactgg atccgacagt gtcgccc                                       27

<210> 4
<211> 52
<212> DNA
<213> Artificial Sequence

<220>
<223> Description of Artificial Sequence:Inner primer(Forward)

<400> 4

tccagtgtcc ctttagtgag ggttaatttc acacaggaaa cagctatgac ca 52

<210> 5
<211> 53
<212> DNA
<213> Artificial Sequence

<220>
<223> Description of Artificial Sequence:Inner primer(Reverse)

<400> 5
gacagtgtcg ccctatagtg agtcgtatta ccagtcacga cgttgtaaaa cga 53

<210> 6
<211> 18
<212> DNA
<213> Artificial Sequence

<220>
<223> Description of Artificial Sequence:Outer primer(Forward)

<400> 6
gtaacgccag ggttttcc 18

<210> 7
<211> 21
<212> DNA

<213> Artificial Sequence

<220>
<223> Description of Artificial Sequence:Outer primer(Reverse)

<400> 7
gaattgtgag cggataacaa t                                                21

<210> 8
<211> 18
<212> DNA
<213> Artificial Sequence

<220>
<223> Description of Artificial Sequence:Loop primer(Forward)

<400> 8
gcgcgcttgg cgtaatca                                                    18

<210> 9
<211> 16
<212> DNA
<213> Artificial Sequence

<220>
<223> Description of Artificial Sequence:Loop primer(Reverse)

<400> 9

gcgcgctcac tggccg                                                    16

<210> 10
<211> 440
<212> DNA
<213> Unknown

<220>
<223> pBluescript II

<400> 10
gagcgcaacg caattaatgt gagttagctc actcattagg caccccaggc tttacacttt 60
atgcttccgg ctcgtatgtt gtgtggaatt gtgagcggat aacaatttca cacaggaaac 120
agctatgacc atgattacgc caagcgcgca attaaccctc actaaaggga acaaaagctg 180
gagctccacc gcggtggcgg ccgctctaga actagtggat cccccgggct gcaggaattc 240
gatatcaagc ttatcgatac cgtcgacctc gagggggggc ccggtaccca attcgcccta 300
tagtgagtcg tattacgcgc gctcactggc cgtcgtttta caacgtcgtg actgggaaaa 360
ccctggcgtt acccaactta atcgccttgc agcacatccc cctttcgcca gctggcgtaa 420
tagcgaagag gcccgcaccg                                                440

<210> 11
<211> 108
<212> DNA
<213> Unknown

<220>
<223> Multiple cloning site from pBluescript II

<400> 11

gagctccacc gcggtggcgg ccgctctaga actagtggat cccccgggct gcaggaattc 60

gatatcaagc ttatcgatac cgtcgacctc gagggggggc ccggtacc 108

<210> 12

<211> 109

<212> DNA

<213> Unknown

<220>

<223> pUC19 109bp Sau3AI flagment(sense)

<400> 12

gatcctttta aattaaaaat gaagttttaa atcaatctaa agtatatatg agtaaacttg 60

gtctgacagt taccaatgct taatcagtga ggcacctatc tcagcgatc 109

<210> 13

<211> 82

<212> DNA

<213> Unknown

<220>

<223> pUC19 82bp Sau3AI flagment(sense)

<400> 13

gatcttttct acggggtctg acgctcagtg gaacgaaaac tcacgttaag ggattttggt 60

catgagatta tcaaaaagga tc 82

<210> 14

<211> 82

<212> DNA

<213> Unknown

<220>

<223> pUC19 82bp Sau3AI flagment(antisense)

<400> 14

gatccttttt gataatctca tgaccaaaat cccttaacgt gagttttcgt tccactgagc 60

gtcagacccc gtagaaaaga tc 82

<210> 15

<211> 45

<212> DNA

<213> Artificial Sequence

<220>

<223> Description of Artificial Sequence:109 oligo: Designed

oligonucleotides (sense) based on pUC19 109bp Sau3AI flagment

<400> 15

gtctgacagt taccaatgct taatcagtga ggcacctatc tcagc 45

<210> 16

<211> 45

<212> DNA

<213> Artificial Sequence

<220>
<223> Description of Artificial Sequence:109 oligo: Designed
oligonucleotides (antisense) based on pUC19 109bp Sau3AI flagment

<400> 16
gctgagatag gtgcctcact gattaagcat tggtaactgt cagac 45

<210> 17
<211> 44
<212> DNA
<213> Artificial Sequence

<220>
<223> Description of Artificial Sequence:Inner primer(Forward)

<400> 17
gtgtcccttt agtgagggtt aatttcacac aggaaacagc tatg 44

<210> 18
<211> 44
<212> DNA
<213> Artificial Sequence

<220>
<223> Description of Artificial Sequence:Inner primer(Reverse)

<400> 18

tgtcgcccta tagtgagtcg tattaccagt cacgacgttg taaa 44

<210> 19

<211> 187

<212> DNA

<213> Unknown

<220>

<223> pUC19

<400> 19

tcaaaaagga tcttcaccta gatcctttta aattaaaaat gaagttttaa atcaatctaa 60

agtatatatg agtaaacttg gtctgacagt taccaatgct taatcagtga ggcacctatc 120

tcagcgatct gtctatttcg ttcatccata gttgcctgac tccccgtcgt gtagataact 180

acgatac 187

<210> 20

<211> 55

<212> DNA

<213> Artificial Sequence

<220>

<223> Description of Artificial Sequence:Inner primer(Forward)

<400> 20

gaccaagttt actcatatat acgacactgg agatcctttt aaattaaaaa tgaag 55

<210> 21
<211> 50
<212> DNA
<213> Artificial Sequence

<220>
<223> Description of Artificial Sequence:Inner primer(Reverse)

<400> 21
tgaggcacct atctcagcga tgacagtgtc acggggagtc aggcaactat 50

<210> 22
<211> 20
<212> DNA
<213> Artificial Sequence

<220>
<223> Description of Artificial Sequence:Outer primer(Forward)

<400> 22
tcaaaaagga tcttcaccta 20

<210> 23
<211> 20
<212> DNA
<213> Artificial Sequence

<220>
<223> Description of Artificial Sequence:Outer primer(Reverse)

<400> 23
gtatcgtagt tatctacacg 20

<210> 24
<211> 19
<212> DNA
<213> Artificial Sequence

<220>
<223> Description of Artificial Sequence:Loop primer(Forward)

<400> 24
actttagatt gatttaaaa 19

<210> 25
<211> 20
<212> DNA
<213> Artificial Sequence

<220>
<223> Description of Artificial Sequence:Loop primer(Reverse)

<400> 25
tctgtctatt tcgttcatcc 20